# EUROPÄISCHE PATENTSCHRIFT

(11) **EP 3 150 169 B1**
(45) Veröffentlichungstag und Bekanntmachung des Hinweises auf die Patenterteilung: **14.03.2018**
(21) Anmeldenummer: 15188045.7
(22) Anmeldetag: 02.10.2015
(51) Int. Cl.: A61F 2/16, G02C 7/04

(54) **MULTIFOKALE LINSE**
MULTIFOCAL LENS
LENTILLE MULTI-FOCALE

(43) Veröffentlichungstag der Anmeldung: 05.04.2017
(73) Patentinhaber: Rayner Intraocular Lenses Limited, Sackville Road Hove East Sussex BN3 7AN (GB)
(72) Erfinder: LUX, Kirsten, 2340 Mödling (AT); PLANK, Nicole, 7212 Forchtenstein (AT); BREZNA, Wolfgang, 2700 Wiener Neustadt (AT); DRAGOSTINOFF, Nikolaus, 1230 Wien (AT)
(74) Vertreter: SR Huebner - Munich Patentanwaltspartnerschaft mbB

(56) Entgegenhaltungen:
- US-A1- 2009 268 155
- US-A1- 2011 234 974

## Beschreibung

Die vorliegende Erfindung betrifft eine multifokale Linse mit einem refraktiven Brennpunkt und einer diffraktiven Struktur, wobei die Struktur in radialer Richtung der Linse, aufgetragen über dem quadrierten Radius, ein periodisches Profil hat, und wobei das Profil pro Periode vier aneinander angrenzende Abschnitte hat, die an ihren Verbindungsstellen nicht differenzierbar sind.

Multifokale Intraokular- oder Kontaktlinsen, d.h. Linsen mit mehreren Brennpunkten, die z.B. für Nah- und Fernsicht (bifokal) oder Nah-, Intermediär- und Fernsicht (trifokal) verwendet werden können, sind seit mehreren Jahrzehnten bekannt und verwenden verschiedenste diffraktive Strukturen auf einer refraktiven Basislinse, um zusätzlich zum refraktiven Brennpunkt einen oder mehrere diffraktive Brennpunkte zu schaffen.

Gemäß den Schriften DE 20 2009 018 881 U1 und EP 2 503 962 B1 werden zur Erzeugung von zwei diffraktiven Brennpunkten Linsen mit diffraktiven Strukturen eingesetzt, deren Profil pro Periode vier abwechselnd monoton steigende und monoton fallende Abschnitte hat, also pro Periode zwei Maxima mit spitzen Winkeln. Eine multifokale Linse gemäß dem Oberbegriff des Anspruchs 1 ist aus Dokument US-A-2009/0268155 bekannt. Die Anmelderin hat erkannt, dass die Einarbeitung solcher Strukturen in die Linse nicht nur zu einer Vielzahl schwierig zu fertigender Profilspitzen führt, sondern auch zu einer suboptimalen Verteilung bzw. Lichtausbeute der Lichtintensitäten in den erzeugten Brennpunkten.

Die Erfindung setzt sich zum Ziel, eine verbesserte Linse zu schaffen, die die Nachteile des Standes der Technik überwindet.

Gemäß der Erfindung wird das Ziel mit einer Linse der einleitend genannten Art erreicht, bei welcher ein erster Abschnitt des Profils monoton fallend und die drei weiteren Abschnitte monoton steigend sind, oder ein erster Abschnitt monoton steigend und die drei weiteren Abschnitte monoton fallend sind, und wobei derjenige weitere Abschnitt, der nicht an den genannten ersten Abschnitt angrenzt, eine größere Steigung hat als die anderen beiden weiteren Abschnitte.

Mit der erfindungsgemäßen Struktur wird eine Linse geschaffen, deren für Nah-, Intermediär- und Fernsicht nutzbare Brennpunkte einen höheren Intensitätsanteil aufweisen als im Stand der Technik bekannt. Zur genaueren Betrachtung des Problems werden im Folgenden diffraktive Brennpunkte "positiver" Ordnung als diejenigen definiert, die zwischen der Linse und ihrem refraktiven Brennpunkt liegen, und diffraktive Brennpunkte "negativer" Ordnung als diejenigen, die auf der der Linse abgewandten Seite des refraktiven Brennpunkts liegen.

Wenn beispielsweise der refraktive Brennpunkt für die Fernsicht eingesetzt wird, entspricht der Brennpunkt erster positiver Ordnung der diffraktiven Struktur einem Abstand für die Intermediärsicht und der Brennpunkt zweiter positiver Ordnung der diffraktiven Struktur einem Abstand für die Nahsicht. Die jeweiligen negativen Brennpunkte der diffraktiven Struktur werden in diesem Fall erst hinter der Netzhaut des Benutzers der Linse abgebildet, weshalb diese für den Benutzer nicht nutzbar sind und zu einer Verschlechterung der Bildqualität beitragen.

Bei der erfindungsgemäßen Linse werden hingegen Intensitätsanteile der (ursprünglich) negativen Ordnungen auf die genutzten positiven Ordnungen bzw. auf die nullte (refraktive) Ordnung abgebildet, wodurch sich im Vergleich zum Stand der Technik ein farbintensiveres und kontrastreicheres Bild ergibt, da die nutzbaren Brennpunkte höhere Intensitätsanteile aufweisen.

Dieselben Vorteile ergeben sich, wenn beispielsweise in einer alternativen Ausführungsform der refraktive Brennpunkt für die Nahsicht eingesetzt wird, und der Brennpunkt erster negativer Ordnung der diffraktiven Struktur einem Abstand für die Intermediärsicht und der Brennpunkt zweiter negativer Ordnung der diffraktiven Struktur einem Abstand für die Fernsicht entspricht. In dieser Ausführungsform sind die positiven Ordnungen der diffraktiven Strukturen schlecht nutzbar, da sie vor dem Nahsicht-Brennpunkt liegen, und die Ordnungen dritter negativer Ordnung gar nicht nutzbar, da sie erst hinter der Netzhaut fokussiert werden. Gemäß der Erfindung werden hier die Intensitätsanteile der positiven Ordnungen auf die nullte (refraktive), negative erste und negative zweite Ordnung abgebildet, wodurch sich im Vergleich zum Stand der Technik wieder eine höhere Lichtausbeute in den nutzbaren Brennpunkten und damit ein farbintensiveres und kontrastreicheres Bild ergibt.

In jeder Ausführungsform hat die erfindungsgemäße Linse überdies den signifikanten Vorteil, dass die diffraktive Struktur der Linse nur ein Maximum pro Periode aufweist und trotzdem zwei diffraktive Brennpunkte erzeugt. Die Herstellung der diffraktiven Struktur auf der Linse kann somit weitaus einfacher und mit geringerem Ausschuss erfolgen, da der Winkel des Maximums größer ist und zudem nur einmal pro Periode auftritt, d.h. nur halb so oft wie bei den diffraktiven Strukturen gemäß dem Stand der Technik, welche zwei diffraktive Brennpunkte erzeugen. Besonders an der Peripherie der Linse, an der die Periodenlängen sehr klein werden, kann durch die dadurch ermöglichte präzisere Fertigung eine höhere Genauigkeit der Linse erreicht werden, was wiederum zu einer genaueren, kontrollierteren Lichtverteilung führt.

Der refraktive Brennpunkt der Linse kann wie erörtert entweder für Nah- oder Fernsicht eingesetzt werden. Wird der refraktive Brennpunkt für die Fernsicht eingesetzt, ist jene Ausführungsform die bevorzugte, bei der der erste Abschnitt monoton fallend und die drei weiteren Abschnitte monoton steigend sind. Alternativ kann der refraktive Brennpunkt für die Nahsicht eingesetzt werden, wobei dann bevorzugt der erste Abschnitt monoton steigend und die drei weiteren Abschnitte monoton fallend sind.

Bevorzugt sind die Abschnitte, aufgetragen über dem quadrierten Radius, linear, d.h. sie ergeben auf der Linse quadratisch ansteigende oder abfallende Flanken. Dies ermöglicht eine einfache Berechnung des Intensitätsverlaufs der Linse. Alternativ können die Abschnitte auch individuelle Verläufe aufweisen, um die Intensitätsverteilung der Linse anzupassen.

In einer bevorzugten Ausführungsform ist der genannte erste Abschnitt im Wesentlichen senkrecht. Unabhängig davon kann auch derjenige weitere Abschnitt, der nicht an den ersten Abschnitt angrenzt, im Wesentlichen senkrecht sein. Beide Maßnahmen ermöglichen ein überaus einfaches Profilmuster, da so nur mehr die Steigung von zwei Abschnitten ermittelt werden muss. Dies erleichtert auch die Fertigung der Linse, weil ein senkrechter Abschnitt, aufgetragen über dem quadrierten Radius, auch eine senkrechte Flanke auf der Linse ergibt.

Um die Berechnung des Profils und in der Folge auch die Fertigung der Linse zu vereinfachen, können jene zwei weiteren Abschnitte, die jeweils an den ersten Abschnitt angrenzen, aufgetragen über dem quadrierten Radius, im Wesentlichen die gleiche Steigung haben.

In einer praktischen Ausführungsform haben jene zwei weiteren Abschnitte, die jeweils an den ersten Abschnitt angrenzen, aufgetragen über dem quadrierten Radius, eine Steigung von 1 µm/mm² bis 10 µm/mm². Weiters beträgt bevorzugt die Periode des Profils, aufgetragen über dem quadrierten Radius, 0,5 mm² bis 1 mm² und die Profiltiefe 2 µm bis 10 µm. Dies liefert eine Linse, deren Brennpunkte für Nah- und Intermediärsicht an von Benutzern gewünschten Entfernungen liegen.

Die Erfindung wird nachfolgend anhand von in den beigeschlossenen Zeichnungen dargestellten Ausführungsbeispielen näher erläutert. In den Zeichnungen zeigen:
Fig. 1 die erfindungsgemäße Linse in einer schematischen Draufsicht;
Fig. 2 die Linse von Fig. 1 in einer schematischen Seitenansicht;
Fig. 3 die Linse von Fig. 1 in einem vergrößerten Halbschnitt;
Fig. 4 das Profil der diffraktiven Struktur der Linse der Fig. 1 - 3, aufgetragen über dem quadrierten Radius der Linse; und
Fig. 5 einen Vergleich der Intensitätsverteilung der erfindungsgemäßen Linse mit jener einer Linse nach dem Stand der Technik.

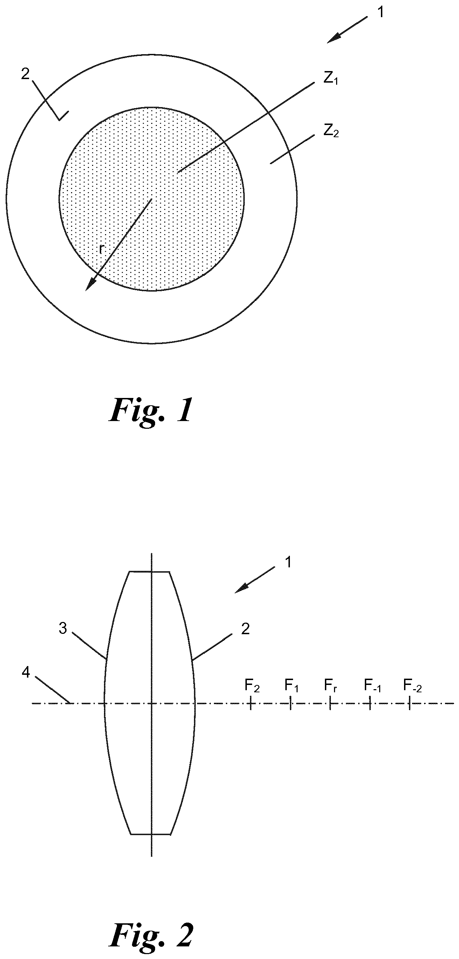

Die Fig. 1 und 2 zeigen eine Linse 1 mit einer Vorderseite 2, einer Rückseite 3 und einer optischen Achse 4. Die Linse 1 hat eine zentrale Zone Z₁ und eine annulare Zone Z₂, die später noch näher erläutert werden. Die beschriebene Linse 1 wird insbesondere als Intraokularlinse oder Kontaktlinse eingesetzt, kann aber auch in optischen Geräten verwendet werden.

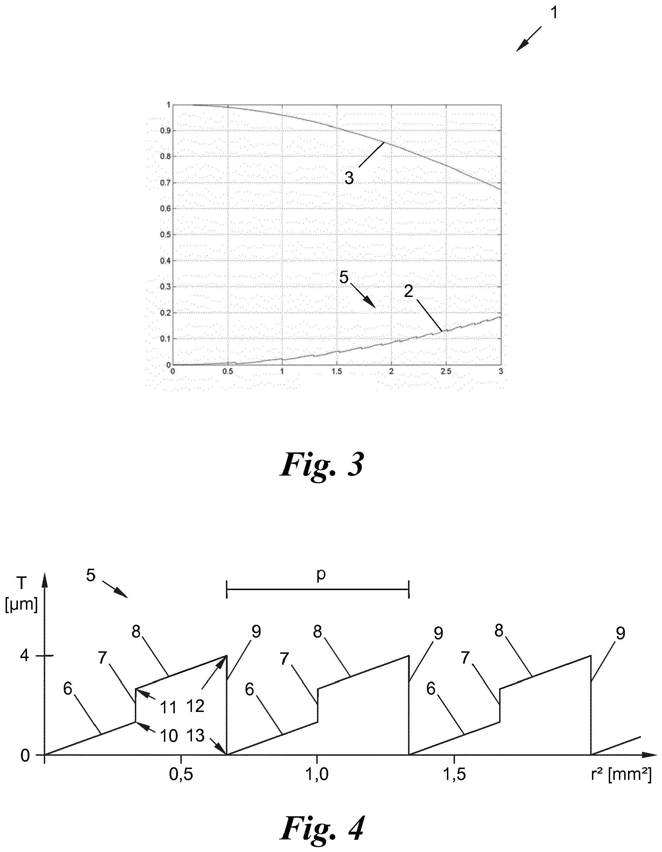

Die Linse 1 hat einen auf der optischen Achse 4 liegenden refraktiven Brennpunkt Fᵣ, der wie unten beschrieben für die Fern- oder Nahsicht eingesetzt werden kann und im Folgenden auch als Brennpunkt nullter Ordnung bezeichnet wird. In die Rück- oder Vorderseite 2, 3 der Linse 1 ist eine diffraktive Struktur 5 eingearbeitet, siehe die Fig. 3 und 4, um die Linse 1 sowohl für Nah- als auch Intermediär- und Fernsicht zu adaptieren.

Die diffraktive Struktur 5 erzeugt eine Vielzahl von auf der optischen Achse 4 liegenden weiteren Brennpunkten Fᵢ, i = ..., -2, -1, 1, 2, ..., die symmetrisch um den refraktiven Brennpunkt Fᵣ verteilt sind, wobei der refraktive Brennpunkt Fᵣ durch die Form der Linse 1 unabhängig von der aufgetragenen diffraktiven Struktur 5 gegeben ist. Die diffraktiven Brennpunkte F₁, F₂ werden als Brennpunkte positiver erster bzw. zweiter Ordnung der diffraktiven Struktur 5 bezeichnet und liegen auf der optischen Achse 4 zwischen der Linse 1 und dem refraktiven Brennpunkt Fᵣ. Die diffraktiven Brennpunkte F₋₁, F₋₂ werden als Brennpunkte negativer erster bzw. zweiter Ordnung der diffraktiven Struktur 5 bezeichnet und liegen auf der der Linse 1 abgewandten Seite des refraktiven Brennpunkts Fᵣ.

Obwohl die (Positions-)Verteilung der Brennpunkte Fᵢ symmetrisch um den refraktiven Brennpunkt Fᵣ ist, soll die Intensitätsverteilung, die den jeweiligen Brennpunkten Fᵢ zukommt, nicht symmetrisch sein. So sollen sich bei einer trifokalen Linse insbesondere drei größte Intensitäten ausbilden, nämlich für die Fern-, Intermediär- und Nahsicht. Dies wird erreicht, indem die diffraktive Struktur 5 gemäß Fig. 4 ausgebildet wird.

Gemäß Fig. 4 (Abszisse: quadrierter Radius r² [mm]; Ordinate: Profiltiefe T [µm]) weist die diffraktive Struktur 5 in radialer Richtung r der Linse 1, aufgetragen über dem quadrierten Radius r², ein periodisches Profil auf, das pro Periode p vier aneinander angrenzende Abschnitte 6, 7, 8, 9 hat, die an ihren Verbindungsstellen 10, 11, 12, 13 nicht differenzierbar sind. Der Ausdruck "aufgetragen über dem quadrierten Radius" bedeutet für die Periodizität, dass die Periodenintervalle p auf der Linse 1 abnehmen.

Die Periode p kann beispielsweise bei einer Intraokular- oder Kontaktlinse im Bereich von 0,5 mm² bis 1 mm² und die Profiltiefe T im Bereich von 2 µm bis 10 µm liegen.

In der Ausführungsform von Fig. 4 ist ein beliebiger "erster" Abschnitt des Profils 5, hier der Abschnitt 9, monoton fallend und die drei weiteren Abschnitte 6, 7, 8 des Profils sind monoton steigend. Der Ausdruck "erster" bezieht sich hierin nicht auf eine Reihenfolge der Abschnitte 6 - 9, sondern dient lediglich zur Unterscheidung von drei "weiteren" Abschnitten. Die Abfolge der Abschnitte 6 - 9 innerhalb einer Periode p kann so frei gewählt oder definiert werden, wodurch beispielsweise jeder der Abschnitte 6, 7, 8, 9 als "Start"-Abschnitt gewählt werden kann bzw. der "erste" Abschnitt 9 nicht notwendigerweise am Beginn der Periode p liegt.

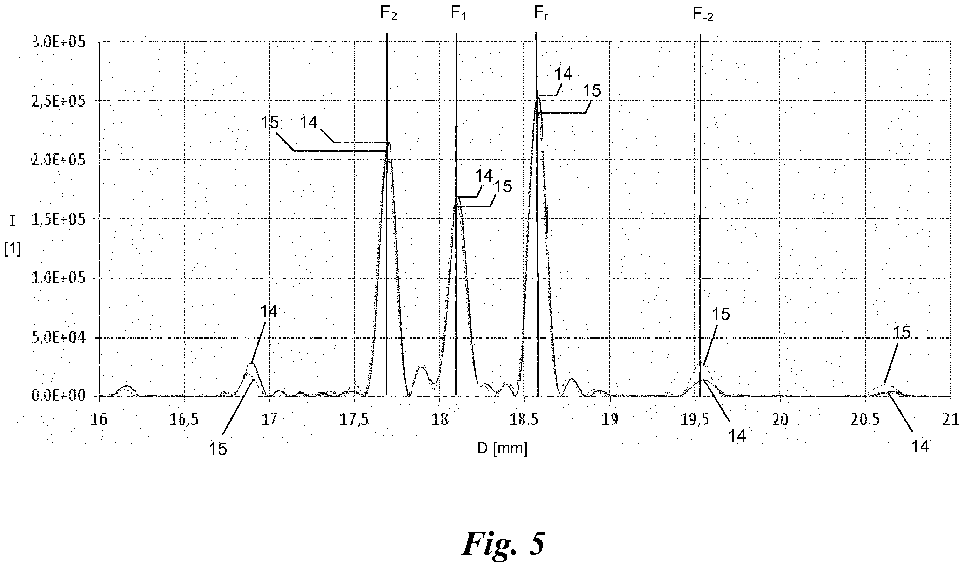

In der in Fig. 4 gezeigten Ausführungsform ist der refraktive Brennpunkt Fᵣ für die Fernsicht ausgelegt und es ergeben sich durch die drei monoton steigenden weiteren Abschnitte 6, 7, 8 und den einen monoton fallenden ersten Abschnitt 9 zwei diffraktive Brennpunkte F₁, F₂ positiver Ordnungen für die Nah- und Intermediärsicht (siehe Fig. 5). Alternativ kann der refraktive Brennpunkt Fᵣ auch z.B. für die Nahsicht ausgelegt werden, wozu dann drei monoton fallende weitere Abschnitte 6, 7, 8 und ein monoton steigender erster Abschnitt 9 eingesetzt werden, die zwei diffraktive Brennpunkte F₋₁, F₋₂ negativer Ordnungen für die Intermediär- und Fernsicht ergeben (nicht gezeigt).

Derjenige weitere Abschnitt, der nicht an den ersten Abschnitt 9 angrenzt, d.h. in Fig. 4 der mittlere weitere Abschnitt 7, hat eine größere Steigung als die anderen beiden weiteren Abschnitte 6, 8. Der Begriff "Steigung" wird hierin als die insgesamt überwundene Steigung eines Abschnitts 6, 7, 8, 9 definiert, d.h. als Steigung zwischen dem Anfangspunkt eines Abschnitts 6, 7, 8 bzw. 9 und dem Endpunkt desselben Abschnitts 6, 7, 8 bzw. 9.

Die Abschnitte 6 - 9 können, aufgetragen über dem quadrierten Radius r², linear sein, wodurch ein monoton steigender Abschnitt 6, 7, 8 auf der Linse 1 eine mit r quadratisch ansteigende Flanke ergibt.

Gemäß Fig. 4 sind zudem der erste Abschnitt 9 und derjenige weitere Abschnitt, der nicht an den ersten Abschnitt 9 angrenzt, d.h. hier der mittlere weitere Abschnitt 7, im Wesentlichen senkrecht, d.h. sie haben eine Steigung von +/- ∞. Alternativ können diese beiden Abschnitte 7, 9 jeweils auch unabhängig voneinander eine endliche Steigung aufweisen (nicht gezeigt).

Jene zwei weiteren Abschnitte 6, 8, die jeweils an den ersten Abschnitt 9 angrenzen, haben aufgetragen über dem quadrierten Radius r² im Wesentlichen die gleiche Steigung. Die Steigung kann beispielsweise bei einer Intraokular- oder Kontaktlinse im Bereich von 1 µm/mm² bis 10 µm/mm² liegen. Die beiden Abschnitte 6, 8 können auch voneinander unterschiedliche Steigungen aufweisen (nicht gezeigt).

Die diffraktive Struktur 5 kann entweder auf die ganze Oberfläche einer Seite 2, 3 der Linse 1 aufgebracht werden oder lediglich in einem zentralen Bereich Z₁ oder einem ringförmigen Bereich Z₂ der Linse 1, wie in Fig. 1 dargestellt. Alternativ oder zusätzlich kann eine Apodisierung der Struktur 5 durchgeführt werden. Dies bedeutet, dass die Profiltiefe T der Struktur 5 mit zunehmendem Linsenradius r abnimmt.

Zur Herstellung der Linse 1 kann die diffraktive Struktur 5 z.B. direkt in einen Linsenrohling eingearbeitet werden, beispielsweise durch Drehen auf einer Drehmaschine. Der Linsenrohling könnte aber auch lediglich ein verarbeitungsfähiges Ausgangsmaterial für einen 3D-Drucker sein, und das Einarbeiten der Struktur in den Linsenrohling erfolgt dann durch 3D-Drucken des Ausgangsmaterials zu der multifokalen Linse 1.

Alternativ könnte die diffraktive Struktur 5 zunächst auch als Negativ in einen Formrohling eingearbeitet, z.B. wieder mittels einer Drehmaschine oder eines 3D-Druckers. Anschließend wird ein Linsenmaterial mit dem Formrohling in Kontakt gebracht, um so die multifokale Linse 1 herzustellen. Das Linsenmaterial kann z.B. bereits zu einem Linsenrohling vorgefertigt sein, in welchen die Struktur 5 mittels des Formrohlings als "Stempel" eingepresst oder eingeprägt wird. Alternativ kann das Linsenmaterial in flüssigem bzw. viskosem Zustand vorliegen und auf den Formrohling gegossen werden, z.B. in einer Form. Daraufhin wird das Linsenmaterial ausgehärtet, z.B. mittels Licht- oder Wärmezufuhr.

Fig. 5 zeigt einen Vergleich des Intensitätsverlaufs 14 (mit ausgezogener Linie dargestellt) der hier vorgestellten Linse 1 mit dem Intensitätsverlauf 15 (mit unterbrochener Linie dargestellt) einer Linse gemäß dem Stand der Technik (Abszisse: Distanz D von der Linse [mm]; Ordinate: Relative Intensität I [1]).

Die für diesen Vergleich eingesetzte Linse 1 mit der hier vorgestellten diffraktiven Struktur 5 hatte eine Periode p, aufgetragen über dem quadrierten Radius r², von 0,65 mm², wobei die Profiltiefe T 4,4 µm betrug. Jene zwei weiteren Abschnitte 6, 8, die jeweils an den ersten Abschnitt 9 angrenzten, hatten - aufgetragen über dem quadrierten Radius r² - eine Steigung von 4,3 µm/mm².

Die Vergleichslinse gemäß dem Stand der Technik hatte im Gegensatz dazu ein periodisches Profil, das innerhalb einer Periode vier Abschnitte hatte, die aufeinanderfolgend monoton steigend, fallend, steigend und fallend waren.

Wie aus dem Diagram von Fig. 5 ersichtlich, ergab sich ein ähnlicher Verlauf der Intensitätsverteilungen im Bereich des refraktiven Brennpunkts Fᵣ. Aus Fig. 5 ist jedoch gut erkennbar, dass die Linse 1 gemäß dem Stand der Technik im Bereich des zweiten negativen Brennpunkts F₋₂ der diffraktiven Struktur 5 größere Intensitätswerte aufwies. Im Gegensatz dazu waren bei der hier vorgestellten Linse 1 nicht nutzbare Intensitäten aus negativen Ordnungen in nutzbare positive Ordnungen verschoben, wie anhand der deutlich erhöhten Intensitäten des Verlaufs 10 an den Brennpunkten F₁ und F₂ sowie der deutlich reduzierten Intensität des Verlaufs 14 am Brennpunkt F₋₂ erkennbar ist. Für den Nutzer der beschriebenen Linse 1 ergibt sich somit ein farbintensiveres und kontrastreicheres Bild als mit Linsen gemäß dem Stand der Technik.

Die Erfindung ist demgemäß nicht auf die dargestellten Ausführungsformen beschränkt, sondern umfasst alle Varianten, Modifikationen und Kombinationen derselben, die in den Rahmen der angeschlossenen Ansprüche fallen.

## Patentansprüche

1. Multifokale Linse mit einem refraktiven Brennpunkt (Fᵣ) und einer diffraktiven Struktur, wobei die Struktur (5) in radialer Richtung (r) der Linse (1), aufgetragen über dem quadrierten Radius (r²), ein periodisches Profil (6, 7, 8, 9) hat, und
wobei das Profil (6, 7, 8, 9) pro Periode vier aneinander angrenzende Abschnitte (6, 7, 8, 9) hat, die an ihren Verbindungsstellen (10, 11, 12, 13) nicht differenzierbar sind,
**dadurch gekennzeichnet, dass** ein erster Abschnitt (9) monoton fallend und die drei weiteren Abschnitte (6, 7, 8) monoton steigend sind, oder ein erster Abschnitt (9) monoton steigend und die drei weiteren Abschnitte (6, 7, 8) monoton fallend sind, und
wobei derjenige weitere Abschnitt (7), der nicht an den genannten ersten Abschnitt (9) angrenzt, eine größere Steigung hat als die anderen beiden weiteren Abschnitte (6, 8).

2. Multifokale Linse nach Anspruch 1, **dadurch gekennzeichnet, dass** die Abschnitte (6, 7, 8, 9), aufgetragen über dem quadrierten Radius (r²), linear sind.

3. Multifokale Linse nach Anspruch 1 oder 2, **dadurch gekennzeichnet**, das der erste Abschnitt (9) im Wesentlichen senkrecht ist.

4. Multifokale Linse nach einem der Ansprüche 1 bis 3, **dadurch gekennzeichnet, dass** derjenige weitere Abschnitt (7), der nicht an den ersten Abschnitt (9) angrenzt, im Wesentlichen senkrecht ist.

5. Multifokale Linse nach einem der Ansprüche 1 bis 4, **dadurch gekennzeichnet, dass** jene zwei weiteren Abschnitte (6, 8), die jeweils an den ersten Abschnitt (9) angrenzen, im Wesentlichen die gleiche Steigung haben.

6. Multifokale Linse nach einem der Ansprüche 1 bis 5, **dadurch gekennzeichnet, dass** jene zwei weiteren Abschnitte (6, 8), die jeweils an den ersten Abschnitt (9) angrenzen, aufgetragen über dem quadrierten Radius (r²), eine Steigung von 1 µm/mm² bis 10 µm/mm² haben.

7. Multifokale Linse nach einem der Ansprüche 1 bis 6, **dadurch gekennzeichnet, dass** die Periode (p) des Profils (6, 7, 8, 9), aufgetragen über dem quadrierten Radius, 0,5 mm² bis 1 mm² und die Profiltiefe (T) 2 µm bis 10 µm beträgt.

8. Multifokale Linse nach einem der Ansprüche 1 bis 7, **dadurch gekennzeichnet, dass** sie eine Intraokularlinse oder Kontaktlinse ist.

## Claims

1. Multifocal lens with a refractive focal point (Fᵣ) and a diffractive structure, the structure (5) having a periodic profile (6, 7, 8, 9) in the radial direction (r) of the lens (1) plotted over the squared radius (r²), and
the profile (6, 7, 8, 9) having four mutually adjacent portions (6, 7, 8, 9) per period which cannot be differentiated at their junctions (10, 11, 12, 13), **characterised in that** a first portion (9) falls monotonically and the three further portions (6, 7, 8) rise monotonically, or a first portion (9) rises monotonically and the three further portions (6, 7, 8) fall monotonically, and
wherein the further portion (7) which does not adjoin said first portion (9) has a steeper gradient than the other two further portions (6, 8).

2. Multifocal lens according to claim 1, **characterised in that** the portions (6, 7, 8, 9) are linear when plotted over the squared radius (r²).

3. Multifocal lens according to claim 1 or 2, **characterised in that** the first portion (9) is substantially vertical.

4. Multifocal lens according to one of claims 1 to 3, **characterised in that** the further portion (7) which does not adjoin the first portion (9) is substantially vertical.

5. Multifocal lens according to one of claims 1 to 4, **characterised in that** the two further portions (6, 8) which each adjoin the first portion (9) have substantially the same gradient.

6. Multifocal lens according to one of claims 1 to 5, **characterised in that** the two further portions (6, 8) which each adjoin the first portion (9) have, plotted over the squared radius (r²), a gradient of 1 µm/mm² to 10 µm/mm².

7. Multifocal lens according to one of claims 1 to 6, **characterised in that** the period (p) of the profile (6, 7, 8, 9), plotted over the squared radius, amounts to 0.5 mm² to 1 mm² and the profile depth (T) to 2 µm to 10 µm.

8. Multifocal lens according to one of claims 1 to 7, **characterised in that** it is an intraocular lens or contact lens.

## Revendications

1. Lentille multifocale avec un point focal réfractif (Fr) et une structure diffractive, la structure (5) dans le sens radial (r) de la lentille (1), rapportée au rayon au carré (r²) ayant un profil périodique (6, 7, 8, 9)
et
le profil (6, 7, 8, 9) ayant par période quatre sections contigües (6 ,7 ,8 ,9), qui ne peuvent pas être différenciées à leurs points de contact (10, 11, 12, 13),
**caractérisée en ce qu'**une première section (9) est à décroissance monotone et les trois autres sections (6, 7, 8) sont à croissance monotone ou bien une première section (9) est à croissance monotone et les trois autres sections (6, 7, 8) sont à décroissance monotone,
ladite section (7) qui n'est pas contigüe à ladite première section (9) ayant une plus grande pente que les deux autres sections (6, 8).

2. Lentille multifocale selon la revendication 1, **caractérisée en ce que** les sections (6, 7, 8, 9), rapportées au rayon au carré (r²), sont linéaires.

3. Lentille multifocale selon une des revendications 1 ou 2, **caractérisée en ce que** la première section (9) est sensiblement verticale.

4. Lentille multifocale selon une des revendications 1 à 3, **caractérisée en ce que** ladite autre section (7), qui n'est pas contigüe à la première section (9), est sensiblement verticale.

5. Lentille multifocale selon une des revendications 1 à 4, **caractérisée en ce que** les deux autres sections (6, 8) qui sont respectivement contigües à la première section (9), ont sensiblement la même pente.

6. Lentille multifocale selon une des revendications 1 ou 5, **caractérisée en ce que** les deux autres sections (6, 8), qui sont respectivement contigües à la première section (9), rapportées au rayon au carré (r²), ont une pente de 1 µm/mm² à 10 µm/mm^{2..}

7. Lentille multifocale selon une des revendications 1 ou 6, **caractérisée en ce que** la période (p) du profil (6, 7, 8, 9), rapportée au rayon au carré, est de 0,5 mm² à 1 mm² et la profondeur du profil (T) est de 2 µm à 10 µm.

8. Lentille multifocale selon une des revendications 1 ou 7, **caractérisée en ce qu'**elle est une lentille intraoculaire ou une lentille de contact.
